# EUROPÄISCHE PATENTSCHRIFT

(11) **EP 0 680 467 B1**
(45) Veröffentlichungstag und Bekanntmachung des Hinweises auf die Patenterteilung: **22.04.1998**
(21) Anmeldenummer: 94905013.2
(22) Anmeldetag: 08.01.1994
(51) Int. Cl.: C07C 269/06, C07C 271/20

(54) **VERFAHREN ZUR HERSTELLUNG C-SUBSTITUIERTER DIETHYLENTRIAMINE**
PROCESS FOR PREPARING C-SUBSTITUTED DIETHYLENE TRIAMINES
PROCEDE DE FABRICATION DE DIETHYLENETRIAMINE A CARBONE SUBSTITUE

(30) Priorität: 25.01.1993 DE 4302289
(43) Veröffentlichungstag der Anmeldung: 08.11.1995
(73) Patentinhaber: SCHERING AKTIENGESELLSCHAFT, 13353 Berlin (DE)
(72) Erfinder: PETROV, Orlin, D-14199 Berlin (DE); HILSCHER, Jean-Claude, D-14195 Berlin (DE); NICKISCH, Klaus, D-12307 Berlin (DE); SCHMITT-WILLICH, Heribert, D-12161 Berlin (DE); GRIES, Heinz, D-10717 Berlin (DE); RADÜCHEL, Bernd, D-13465 Berlin (DE); PLATZEK, Johannes, D-14195 Berlin (DE)
(86) Internationale Anmeldenummer: EP9400034
(87) Internationale Veröffentlichungsnummer: WO9417033

(56) Entgegenhaltungen:
- EP-A- 0 405 704
- EP-A- 0 466 200
- WO-A-91/14459
- DE-B- 1 155 122
- US-A- 2 364 178
- US-A- 3 236 895

## Beschreibung

Die Erfindung betrifft den in den Patentansprüchen gekennzeichneten Gegenstand, d.h. ein Verfahren zur Herstellung C-substituierter Diethylentriamine.

In der Literatur werden verschiedene Verfahren zur Synthese C-substituierter Diethylentriamine beschrieben, die alle nach dem gleichen Grundschema ablaufen [siehe z.B. Inorganic Chem. 25 (1986) 2772; DE 37 10 730; U.S. 4,622,420; Nucl. Med. Biol. (1991) 313; WO 91/14459]. Dabei wird ein Aminosäureester mit freier oder geschützter Aminogruppe mit Ethylendiamin zum entsprechenden Amid umgesetzt. Anschließend wird das Amid mit Diboran zum Amin reduziert, wobei gegebenenfalls vorhandene Schutzgruppen zuvor abzuspalten sind.
Diese Methode ist mit den folgenden Nachteilen behaftet:
a) Es ist ein großer Boran-Überschuß (5-10 Moläquivalent) notwendig, da das zu reduzierende Amid sekundär ist und noch zwei freie Amino-Gruppen enthält.
b) Die Zwischenstufen sind schlecht kristallisierbar und schwer zu reinigen.
c) Wegen der Giftigkeit und des hohen Preis des Borans ist dieses Verfahren für die großtechnische Anwendung nicht geeignet oder zumindest wegen des zu betreibenden hohen Sicherheitsaufwands ökonomisch unrentabel.
d) Es werden sehr lange Reaktionszeiten sowie hohe Reaktionstemperaturen benötigt (18-30h in der Siedehitze).

Versuche, das Diboran durch andere besser handhabbare Reduktionsmittel wie z.B. Lithiumaluminiumhydrid (LAH) oder Diisobutylaluminiumhydrid (DIBAH) zu ersetzen scheiterten. So entstehen im Fall von LAH komplexe Reaktionsgemische. Aber auch DIBAH ist nicht universell anwendbar, so werden z.B. gegebenfalls im Molekül enthaltene Phenolethergruppen (als C-Substituent) abgespalten.

Es besteht daher nach wie vor ein großes Interesse an einem technisch praktikablen, universell anwendbarem Verfahren zur Herstellung C-substituierter Diethylentriamine, um so mehr als die besagten Substanzen wichtige Edukte zur Herstellung derivatisierter Diethylentriaminpentaessigsäuren sind, die ebenso wie die Diethylentriamine selbst, wichtige Substanzklassen in der pharmazeutischen Industrie sind [siehe z.B. U.S. 4,622,420; Inorg. Chem. 25 (1986) 2772; EP 0 405 704; Nucl. Mol. Biol. (1991) 31].

So sind insbesondere Metallkomplexe der Pentaessigsäure-Derivate wichtige Verbindungen zur Herstellung von Kontrastmitteln im Bereich der Diagnostik (EP 0 405 704).

Aufgabe der Erfindung ist es daher, ein allgemein anwendbares Verfahren zur Herstellung C-substituierter Diethylentriamine bereitzustellen, daß die Nachteile des Standes der Technik überwindet und insbesondere ohne die Verwendung des teuren und giftigen Diborans auskommt.

Diese Aufgabe wird durch das erfindungsgemäße Verfahren zur Herstellung Kohlenstoff-substituierter Diethylentriamine der allgemeinen Formel I gelöst, 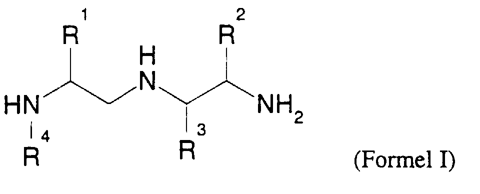 worin
R¹ für eine Gruppe -(CH₂)ₘ-(C₆H₄)_{q}-(O)ₖ-(CH₂)ₙ-(C₆H₄)ₗ-(O)ᵣ-R oder für eine Gruppe -(CH₂)ₘ-(C₆H₁₀)_{q}-(O)ₖ-(CH₂)ₙ-(C₆H₁₀)ₗ-(O)ᵣ-R steht,
   worin m und n unabhängig voneinander die Ziffern 0-5 bedeuten,
   k, l, q und r unabhängig voneinander die Ziffern 0 oder 1 bedeuten,
   R für ein Wasserstoffatom, eine Schutzgruppe einen gegebenenfalls OR⁵-substituierten C₁-C₆-Alkylrest oder eine CH₂-COOR⁵ -Gruppe stehen,
   mit R⁵ in der Bedeutung eines Wasserstoffatoms, eines C₁-C₆-Alkylrestes oder einer Benzylgruppe,
   unter der Maßgabe, daß eine direkte Sauerstoff-Sauerstoff-Bindung nicht zugelassen ist,
R² und R³ jeweils für ein Wasserstoffatom stehen oder gemeinsam eine -(CH₂)ₚ-Alkylenbrücke bilden,
   worin p die Ziffern 3 oder 4 bedeutet und
R⁴ für eine Aminoschutzgruppe, bevorzugt für eine Benzyloxycarbonylgruppe steht, welches **dadurch gekennzeichnet** ist, daß ein am Stickstoff geschützter Aminoethylalkohol der allgemeinen Formel II 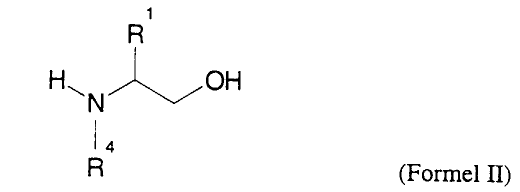 worin R¹ und R⁴ die angegebenen Bedeutungen haben,
   mit Methansulfonsäurechlorid, Tosylchlorid oder Trifluoressigsäureanhydrid in einem organischen Lösungsmittel, unter Zugabe einer Base, zum entsprechenden Mesylat, Tosylat oder Triflat umgesetzt wird, anschließend filtriert wird und das Filtrat mit einem Ethylendiamin der allgemeinen Formel III 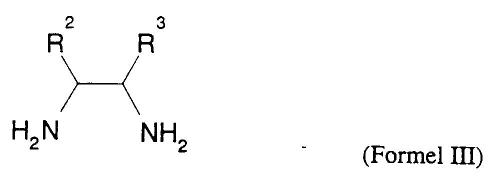 worin R² und R³ die angegebenen Bedeutungen haben
   zu dem gewünschten Stickstoff-geschützten, Kohlenstoff-substituierten Triamin der allgemeinen Formel I umgesetzt wird.

Das erfindungsgemäße Verfahren geht also von leicht zugänglichen Stickstoff-geschützten Aminoethylalkoholen aus, die zunächst mit Methansulfonsäurechlorid, Toluolsulfonsäurechlorid oder Trifluoressigsäureanhydrid in einem nicht protischen organischen Lösungsmittel wie z.B. THF, Diethylether oder Dioxan unter Zugabe einer Base, bevorzugt Triethylamin, umgesetzt werden. Dabei werden neben den 2-Aminoethanol-Alkoholgruppen auch weitere gegebenenfalls im Rest R¹ enthaltend OH-Gruppen zum entsprechenden Mesylat, Tosylat bzw. Triflat umgewandelt. Diese können gewünschtenfalls in einem späteren Reaktionsschritt nach dem Fachmann bekannten Methoden wieder abgespalten oder falls gewünscht umgewandelt werden.

Die so erhaltenen Mesylate, Tosylate bzw. Triflate werden direkt mit Ethylendiamin, 1,2-Diaminocyclopentan bzw. 1,2-Diaminocyclohexan zu den entsprechenden monogeschützten Triaminen umgesetzt. Eine vorherige Isolierung bzw. Reinigung der Mesylate (Tosylate, Triflate) ist dabei nicht erforderlich.

Die als Ausgangssubstanz benötigten N-geschützten Aminoethylalkohole der allgemeinen Formel II sind in der Fachmann bekannten Weise [Bull. Chem. Soc. Japan (1984) 2327] durch Reduktion eines N-geschützten Aminosäureesters der allgemeinen Formel IV 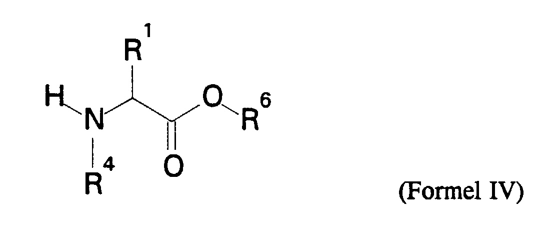 worin
R¹ und R⁴ die angegebenen Bedeutungen haben und
R⁶ für einen geradkettigen oder verzweigten C₁-C₆-Alkylrest steht mit NaBH₄ in einem organischen Lösungsmittel leicht zugänglich.

Anstelle des NaBH₄ kann als Reduktionsmittel auch LiBH₄ (J. Org. Chem. (1982) 1604] bzw. NaBH₄ mit einem Zusatz von LiBr (Tetrahedron Letters (1988) 4919] verwendet werden.

Sofern gewünscht kann die Stickstoffschutzgruppe der Kohlenstoff-substituierten Diethylentriamine der allgemeinen Formel I in der Fachmann bekannten Weise abgespalten werden (T. Green "Protective Groups in Organic Synthesis, Wiley (1981) 239). Zweckmäßigerweise reinigt man dazu die Kohlenstoff-substituierten Diethylentriamine der allgemeinen Formel I durch Ansäuern mit Salzsäure und Überführung in die entsprechenden Hydrochloride. Diese werden dann aus einem geeigneten organischen Lösungsmittel, wie z.B. Alkoholen, bevorzugt Methanol, umkristllisiert. Anschließend hydriert mandie Aminoschutzgruppe R⁴ unter Verwendung eines geeigneten Pd-Katalysators ab. Als Reaktionsmedien eignen sich ebenfalls Alkohole. Der Katalysator wird nach erfolgter Hydrogenolyse abfiltriert und anschließend wird das Salz aus dem Filtrat auskristallisiert.
Das Amin kann gewünschtenfalls aus dem Kristallisat (Hydrochlorid) durch Zugabe einer anorganischen Base, bevorzugt NaOH, freigesetzt werden.

Das erfindungsgemäße Verfahren vermeidet den Einsatz des giftigen Diborans, das Produkt kann aufgrund des guten Kristallisationsverhaltens der Zwischenstufen in hoher Reinheit und Ausbeute erhalten werden. So verläuft die Alkylierung der Diamine mit den entsprechenden Mesylaten, Tosylaten bzw. Triflaten nicht nur in besserer Ausbeute als bei der konventionellen Synthese (Boranreduktion) sondern auch in besserer Ausbeute und höherer Selektivität als bei der bereits vorbekannten Monoalkylierung von Ethylendiamin mit einfachen Alkylhalogeniden, wie sie z.B. im J. Am. Chem. Soc. 67 (1945) 1531 oder in der EP 0 466 200 beschrieben ist. Als weitere Vorteile gegenüber den in EP 0466200 und US 3236895 angegebenen Verfahren sind das Vermeiden von N-Schutzgruppen im Ethylendiamin der allgemeinen Formel III sowie der Isolierung bzw. Reinigung der Mesylate (Tosylate, Triflate) zu nennen.

Wegen der prinzipiellen Unterschiede des erfindungsgemäßen Verfahrens und des Verfahrens über die Boranreduktion, ist ein Ausbeutevergleich nur über den gesamten Syntheseweg, d.h. bezogen auf gleiche Anfangs- und Endprodukte sinnvoll und möglich.

So werden für die Synthese des (S)-1-(4-Ethoxybenzyldiethylentriamins) aus dem (S)-N-Benzyloxycarbonyl-O-ethyltyrosinmethylester bei dem erfindungsgemäßen Verfahren über 80% Gesamtausbeute erreicht (Beispiel 1a-d), wohingegen bei dem Verfahren über das entsprechende Amid, gefolgt von der Abspaltung des Benzyloxycarbonyl-Restes und einer Reduktion mit Diboran, die Gesamtausbeute lediglich bei 60% liegt.

Die Erfindung wird durch die nachfolgenden Beispiele erläutert, ohne sie auf diese beschränken zu wollen.

### Beispiel 1

a) N-Benzyloxycarbonyl-O-ethyltyrosinmethylester
32,94 g (100 mmol) N-Benzyloxycarbonyltyrosinmethylester werden in 200 ml DMF mit 27,64 g (200 mmol) gemahlenem Kaliumcarbonat versetzt. Zu dieser Suspension werden 8,96 ml (110 mmol) lodethan zugetropft und über Nacht bei Raumtemperatur gerührt. Die Lösung wird eingeengt, zwischen Essigester und Wasser verteilt und die organische Phase nach Trocknen (Na₂SO₄) mit Hexan versetzt. Die Titelverbindung kristallsiert aus.
Ausbeute: 32,88 g (92 %)
Fp.: 50-56°C

| Analyse: | | | |
|---|---|---|---|
| ber. | C 67,21 | H 6,49 | N 3,92 |
| gef. | C 66,96 | H 6,57 | N 3,81 |

b) (S)-N-Benzyloxycarbonyl-2-(4-ethoxybenzyl)-2-aminoethanol
Zu einer Lösung von 221.41 g (605.9 mmol) (S)-N-Benzyloxycarbonyl-O-ethyltyrosinmethylester in 1.5 1 Tetrahydrofuran werden bei Raumtemperatur 31.80 g (848.4 mmol) Natriumborhydrid zugegeben. Dazu werden unter Rühren 270 ml Methanol innerhalb 2 Stunden zugetropft. Anschließend wird das Tetrahydrofuran im Vakuum abdestilliert, der Rest in 1 1 Wasser aufgenommen und dreimal mit 700 ml Essigester extrahiert. Die vereinigte organische Phase wird mit Wasser gewaschen, mit Natriumsulfat getrocknet und aufkonzentriert. Es wird aus Essigester/Hexan umkristallisiert.
Ausbeute: 187,0 g (93,7 %)
Schmelzpunkt: 112-117°C

| Analyse: | | | |
|---|---|---|---|
| ber. | C 69,28 | H 7,04 | N 4,25 |
| gef. | C 68,93 | H 7,27 | N 3,96 |

c) (S)-N-Benzyloxycarbonyl-N'-(2-aminoethyl)-1-(4-ethoxybenzyl)ethylendiamin
Zu einer Lösung von 84,00 g (255,0 mmol) (S)-N-Benzyloxycarbonyl-2-(4-ethoxybenzyl)-2-aminoethanol und 37.82 ml (272,9 mmol) Triethylamin in 330 ml Tetrahydrofuran werden bei 4°C langsam unter Rühren 20,86 ml (267,8 mmol) Methansulfonylchlorid zugetropft. Nach 2 Stunden wird der ausgefallene Niederschlag abfiltriert und das Filtrat mit 427,0 ml (6,4 mol) Ethylendiamin versetzt. Die Lösung wird 4 Stunden bei 50°C gerührt und anschließend im Vakuum konzentriert. Der Rückstand wird mit Wasser aufgenommen, mit Ethylacetat extrahiert, mit Natriumsulfat getrocknet und am Rotationsverdampfer konzentriert. Der Rest wird in Methanol aufgenommen und bei 0°C mit konz. Salzsäure angesäuert. Nach Absaugen und Trocknen wird das Produkt in Form farbloser Kristalle als Dihydrochlorid erhalten.
Ausbeute: 95,7 g (84,5 %)
Schmelzpunkt: 223-225°C (Zers.)

| Analyse: | | | | |
|---|---|---|---|---|
| ber. | C 56,76 | H 7,03 | N 9,46 | Cl 15,96 |
| gef. | C 55,34 | H 6,94 | N 9,45 | Cl 16,32 |

¹H-NMR (CDCl₃, freie Base): 7,45 - 7,29 m [5]; 7,15 d, J = 8 Hz, [2]; 6,80 d, J = 8 Hz, [2]; 5,25 br. d [1]; 5,08 ABq [2]; 3,98 q, J = 6 Hz, [2]; 3,90 m [1]; 2,90 - 2,55 m [8]; 1,50 br. s [3]; 1,40 tr, J = 6 Hz [3].
d) (S)-1-(4-Ethoxybenzyl)diethylentriamin
90,0 g (202,5 mmol) (S)-N-Benzyloxycarbonyl-N'-(2-aminoethyl)-1-(4-ethoxybenzyl)-ethylendiamindihydrochlorid werden in 2,7 1 Methanol suspendiert und nach Zugabe von 7,5 g 10 % Pd/C 1 Stunde bei 15 bar hydriert. Anschließend wird der Katalysator abfiltriert und das Filtrat auf 150 ml aufkonzentriert. Die ausgefallenen Kristalle werden abgesaugt.
Ausbeute: 56,86 g (90,5 %)
Schmelzpunkt: 227 - 231 °C (Zers.)

| Analyse: | | | | |
|---|---|---|---|---|
| ber. | C 50,33 | H 8,12 | N 13,54 | Cl 22,85 |
| gef. | C 50,71 | H 8,32 | N 13,77 | Cl 23,27 |

Zur Freisetzung des Triamins werden 14,7 g (366 mmol) pulverisierte NaOH zu einer Suspension der so gewonnenen Kristalle in 100 ml Methanol zugegeben. Das ausgefallene Natriumchlorid wird abfiltriert und das Filtrat eingeengt. Man erhält nach Trocknen bei 50°C im Vakuum 43,5 g (100 %) eines farblosen Öls.

### Beispiel 2

a) (S)-N-Benzyloxycarbonyl-2-(4-hydroxybenzyl)-2-aminoethanol
Zu einer Lösung von 50,0 g (151 mmol) (S)-N-Benzyloxycarbonyltyrosinmethylester in 0,5 1 Tetrahydrofuran werden bei Raumtemperatur 15,75 g (400 mmol) Natriumborhydrid gegeben. Dazu werden unter Rühren 100 ml Methanol innerhalb von 2 Stunden getropft. Das Reaktionsgemisch wird 2 Stunden bei 50 °C gerührt. Anschließend wird das Tetrahydrofuran im Vakuum abdestilliert, der Rest in 400 ml Wasser aufgenommen und dreimal mit je 300 ml Essigester extrahiert. Die vereinigten organischen Phasen werden mit Wasser gewaschen, mit Natriumsulfat getrocknet und aufkonzentriert. Es wird aus Essigester/Hexan umkristallisiert.
Ausbeute: 41,5 g (91 %)
Schmelzpunkt: 79-82,5°C

| Analyse: | | | |
|---|---|---|---|
| ber. | C 67,76 | H 6,36 | N 4,65 |
| gef. | C 68,12 | H 6,37 | N 4,73 |

b) (S)-N-Benzyloxycarbonyl-N'-(2-aminoethyl)-1-(4-methylsulfonyloxybenzyl)ethylendiamin
Zu einer Lösung von 7,3 g (24,2 mmol) (S)-N-Benzyloxycarbonyl-2-(4-hydroxybenzyl)-2-aminoethanol und 7,35 g (72,7 mmol) Triethylamin in 30 ml Tetrahydrofuran werden bei 4 °C langsam unter Rühren 7,14 g (60,6 mmol) Methansulfonylchlorid getropft. Nach 2 Stunden wird der ausgefallene Niederschlag abfiltriert und das filtrat mit 29,1 g mmol) Ethylendiamin versetzt. Die Lösung wird 4 Stunden bei 50 °C gerührt und anschließend im Vakuum konzentriert. Der Rückstand wird in Wasser aufgenommen Ethylacetat extrahiert, mit Natriumsulfat getrocknet und am Rotationsverdampfer konzentriert. Der Rest wird in Methanol aufgenommen und bei 0 °C mit konzentriert Salzsäure angesäuert. Nach Absaugen und Trocknen wird das Produkt in Form farblc Kristalle als Dihydrochlorid erhalten.
Ausbeute: 9,4 g (79 %)
Schmelzpunkt: 216-218 °C (Zers.)

| Analyse: | | | | |
|---|---|---|---|---|
| ber. | C 48,58 | H 5,91 | N 8,50 | Cl 14,34 |
| gef. | C 48,71 | H 6,32 | N 8,74 | Cl 13,97 |

¹H-NMR (CDCl_{3,} freie Base): 7,4 - 7,15 m [9]; 5,2 br, d, J = 9 Hz, [1]; 5,08 s [2]; 3,94 m [1]; 3,14 s [3]; 2,95-2,60 m [8]; 1,52 br. s [3].
c) (S)-1-(4-Methylsulfonyloxybenzyl)diethylentriamin
5,0 g (10,1 mmol) (S)-N-Benzyloxycarbonyl-N'-(2-aminoethyl)-1-(4-methylsulfonyloxybenzyl)ethylendiamindihydrochlorid werden in 50 ml Methanol suspendiert und nach Zugabe von 0,84 g 10% Pd/C-Katalysator 4 Stunden bei 15 bar hydriert. Anschließend wird der Katalysator abfiltriert, das Filtrat mit 1 ml konz. Salzsäure versetzt und auf 20 ml eingeengt. Die ausgefallenen Kristalle werden abgesaugt.
Ausbeute: 3,6 g (90 %)
Schmelzpunkt: 226-230 °C

| Analyse: | | | | |
|---|---|---|---|---|
| ber. | C 36,33 | H 6,10 | N 10,59 | Cl 26,81 |
| gef. | C 36,71 | H 6,32 | N 10,73 | Cl 26,27 |

Zur Freisetzung des Triamins werden 1,1 g (27,3 mmol) pulverisiertes Natriumhydroxid zu einer Suspension der so gewonnen Kristalle in 10 ml Methanol gegeben. Das ausgefallene Natriumchlorid wird abfiltriert und das Filtrat eingeengt. Man erhält nach Trocknen bei 50 °C im Vakuum 2,6 g (100%) eines farblosen Öls.

### Beispiel 3

### N-(2-Benzyloxycarbonylamino-3-phenylpropyl)-cyclopentyl- 1,2-diamin-dihydrochlorid

Zu einer Lösung von 5,7 g (20 mmol) N-Benzyloxycarbonylphenylalaninol [Correa et al. Synth. Commun. 21, 1-9 (1991] und 3,0 ml (21,7 mmol) Triethylamin in 30 ml Tetrahydrofuran werden bei 4°C langsam unter Rühren 1,64 ml (21 mmol) Methansulfonylchlorid zugetropft. Nach 2 Stunden wird der ausgefallene Niederschlag abfiltriert und das Filtrat zu 50,1 g (500 mmol) 1,2-Diaminocyclopentan [Jaeger und Blumendal], Z. anorg. Chem. 175, 161 (1928)] zugetropft. Die Lösung wird 4 Stunden bei 50°C gerührt und anschließend im Vakuum konzentriert. Der Rückstand wird mit Wasser aufgenommen, mit Ethylacetat extrahiert, mit Natriumsulfat getrocknet und am Rotationsverdampfer eingeengt. Das verbleibende Öl wird in Methanol aufgenommen und bei 0°C mit konz. Salzsäure angesäuert. Nach Absaugen und Trocknen wird das Produkt in amorpher Form erhalten.
Ausbeute: 7,1 g (81 %)

| Analyse: | | | | |
|---|---|---|---|---|
| ber. | C 60,00 | H 7,09 | Cl 16,10 | N 9,54 |
| gef. | C 59,43 | H 7,20 | Cl 16,61 | N 9,29 |

### Beispiel 4

### N-(2-Benzyloxycarbonylamino-4-methylpentyl)-cyclohexyl- 1,2-diamin-dihydrochlorid

Zu einer Lösung von 5,0 g (20 mmol) N-Benzyloxycarbonyl-leucinol [Correa et al. Synth. Commun. 21, 1-9 (1991)] und 3,0 ml (21,7 mmol) Triethylamin in 30 ml Tetrahydrofuran werden bei 4°C langsam unter Rühren 1,64 ml (21 mmol) Methansulfonylchlorid zugetropft. Nach 2 Stunden wird der ausgefaliene Niederschlag abfiltriert und das Filtrat zu 120 ml (1 mol) trans-1,2-Diaminocyclohexan zugetropft. Die Lösung wird 4 Stunden bei 50°C gerührt und anschließend im Vakuum konzentriert. Der Rückstand wird mit Wasser aufgenommen, mit Ethylacetat extrahiert, mit Natriumsulfat getrocknet und am Rotationsverdampfer eingeengt. Das verbleibende Öl wird in Methanol aufgenommen und bei 0°C mit konz. Salzsäure angesäuert. Nach Absaugen und Trocknen wird das Produkt in amorpher Form erhalten.
Ausbeute: 7,1 g (84 %)

| Analyse: | | | | |
|---|---|---|---|---|
| ber. | C 57,14 | H 8,39 | Cl 16,87 | N 9,99 |
| gef. | C 57,43 | H 8,20 | Cl 16,22 | N 9,69 |

### Beispiel 5

a) N-Benzyloxycarbonyl-O-methyl-tyrosinmethylester
32,94 g (100 mmol) N-Benzyloxycarbonyl-tyrosinmethylester werden in 200 ml DMF mit 27,64 g (200 mmol) gemahlenem Kaliumcarbonat versetzt. Zu dieser Suspension werden 15,6 g (110 mmol) Iodmethan zugetropft und über Nacht bei Raumtemperatur gerührt. Die Lösung wird eingeengt, zwischen Essigester und Wasser verteilt und die organische Phase nach Trocknen (Na₂SO₄) mit Hexan versetzt. Die Titelverbindung kristallisiert aus.
Ausbeute: 31,9 g (93 %)

| Analyse: | | | |
|---|---|---|---|
| ber. | C 66,46 | H 6,16 | N 4,08 |
| gef. | C 66,60 | H 6,23 | N 3,99 |

b) Nₐ-Benzyloxycarbonyl-O-methyl-tyrosin-(2-aminocyclohexyl)-amid-hydrochlorid
24,0 g (70 mmol) N-Benzyloxycarbonyl-O-methyl-tyrosinmethylester werden in 50 ml Methanol gelöst und in ca. 2 Stunden in 420 ml (3,5 mol) trans-1,2-Diaminocyclohexan eingetropft. Die Lösung wird 24 Stunden bei Raumtemperatur gerührt und anschließend im Ölvakuum zur Trockne eingedampft. Der ölige Rückstand wird in Essigester aufgenommen und mehrmals zur Beseitigung von Diaminocyclohexan-Resten mit Wasser ausgeschüttelt. Die organische Phase wird getrocknet (Na₂SO₄) und mit 2N Chlorwasserstoff in Essigester versetzt. Der nach kurzer Zeit entstehende Niederschlag wird abfiltriert und bei 50°C im Vakuum getrocknet.
Ausbeute: 23,0 g (71 %)

| Analyse: | | | | |
|---|---|---|---|---|
| ber.: | C 62,40 | H 6,98 | Cl 7,67 | N 9,10 |
| gef.: | C 61,70 | H 7,05 | Cl 7,38 | N 9,25 |

c) N- [2-Amino-3-(4-Methoxyphenyl)propyl]-cyclohexan- 1,2-diamin-trihydrochlorid
18,5 g (40 mmol) Nₐ-Benzyloxycarbonyl-O-methyl-tyrosin-(2-aminocyclohexyl)-amid-hydrochlorid werden in 200 ml Methanol suspendiert und unter Stickstoff mit Palladium auf Aktivkohle (10 % Pd) versetzt und wahlweise im Autoklaven oder unter Normaldruck mit Wasserstoff hydriert. Nach beendeter Reaktion (ca. 2-6 Stunden) wird vom Katalysator abgesaugt und das Filtrat eingeengt. Das erhaltene Öl wird in 320 ml 1 M Diboran/Tetrahydrofuran-Komplex-Lösung (320 mmol) suspendiert und 48 Stunden unter Rückfluß gerührt. Anschließend wird im Eisbad gekühlt und die Reaktion durch Zugabe von 15 ml Methanol beendet. Man läßt eine Stunde im Eisbad rühren und leitet dann Chlorwasserstoff ein, wobei das Trihydrochlorid des gewünschten Amins ausfällt. Der Niederschlag wird abgesaugt und über P₂O₅ getrocknet.
Ausbeute: 14,2 g (92 %)

| Analyse: | | | | |
|---|---|---|---|---|
| ber.: | C 49,68 | H 7,82 | Cl 27,50 | N 10,86 |
| gef.: | C 49,21 | H 7,70 | Cl 28,75 | N 10,20 |

## Patentansprüche

1. Verfahren zur Herstellung Kohlenstoff-substituierter Diethylentriamine der allgemeinen Formel I, 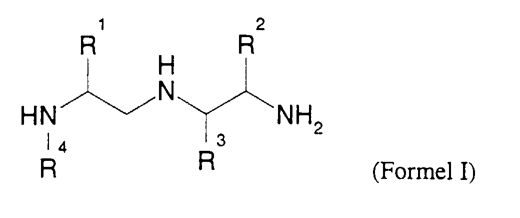 worin
R¹ für eine Gruppe -(CH₂)ₘ-(C₆H₄)_{q}-(O)ₖ-(CH₂)ₙ-(C₆H₄)ₗ-(O)ᵣ-R oder für eine Gruppe -(CH₂)ₘ-(C₆H₁₀)_{q}-(O)ₖ-(CH₂)ₙ-(C₆H₁₀)ₗ-(O)ᵣ-R steht,
worin m und n unabhängig voneinander die Ziffern 0-5 bedeuten,
k, l, q und r unabhängig voneinander die Ziffern 0 oder 1 bedeuten,
R für ein Wasserstoffatom, eine Schutzgruppe, einen gegebenenfalls OR⁵-substituierten C₁-C₆-Alkylrest oder eine CH₂-COOR⁵ -Gruppe stehen,
mit R⁵ in der Bedeutung eines Wasserstoffatoms, eines C₁-C₆-Alkylrestes oder einer Benzylgruppe,
unter der Maßgabe, daß eine direkte Sauerstoff-Sauerstoff-Bindung nicht zugelassen ist,
R² und R³ jeweils für ein Wasserstoffatom stehen oder gemeinsam eine -(CH₂)ₚ-Alkylenbrücke bilden,
worin p die Ziffern 3 oder 4 bedeutet und
R⁴ für eine Aminoschutzgruppe steht,
**dadurch gekennzeichnet**, daß ein am Stickstoff-geschützter Aminoethylalkohol der allgemeinen Formel II 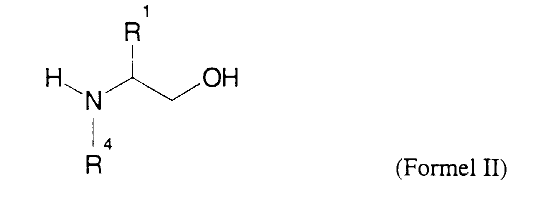 worin R¹ und R⁴ die angegebenen Bedeutungen haben,
mit Methansulfonsäurechlorid, Tosylchlorid oder Trifluoressigsäureanhydrid in einem organischen Lösungsmittel, unter Zugabe einer Base, zum entsprechenden Mesylat, Tosylat oder Triflat umgesetzt wird, anschließend filtriert wird und das Filtrat mit einem Ethylendiamin der allgemeinen Formel III 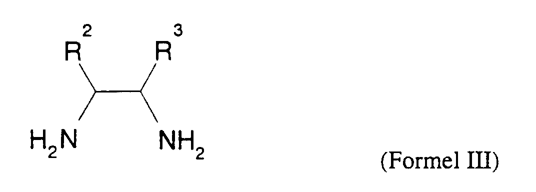 worin R² und R³ die angegebenen Bedeutungen haben
zu dem gewünschten Stickstoff-geschützten, Kohlenstoff-substituierten Triamin der allgemeinen Formel I umgesetzt wird.

2. Verfahren nach Anspruch 1, dadurch gekennzeichnet, daß die Gruppe -(CH₂)ₘ-(C₆H₄)_{q}-(O)ₖ-(CH₂)ₙ-(C₆H₄)ₗ-(O)ᵣ-R für einen Isobutyl-, Benzyl-, 4-Ethoxybenzyl- oder einen 4-Methoxybenzyl-Rest steht.

3. Verfahren nach Anspruch 1, dadurch gekennzeichnet, daß als Aminoschutzgruppe R⁴ ein Benzyloxycarbonylrest ist.

4. Verfahren nach Anspruch 1, dadurch gekennzeichnet, daß als Base Triethylamin eingesetzt wird.

5. Verfahren nach Anspruch 1, dadurch gekennzeichnet, daß der Stickstoff-geschützte Aminoalkohol der Formel II mit Methansulfonsäurechlorid umgesetzt wird.

6. Verfahren nach Anspruch 1, dadurch gekennzeichnet, daß der Stickstoff-geschützte Aminoalkohol der Formel II mit Toluolsulfonsäurechlorid umgesetzt wird.

## Claims

1. Process for the manufacture of carbon-substituted diethylenetriamines of the general formula I 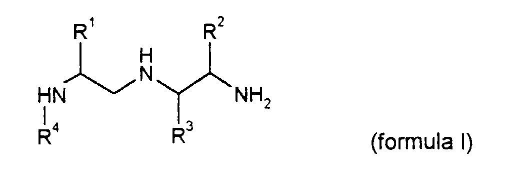 wherein
R¹ represents a -(CH₂)ₘ-(C₆H₄)_{q}-(O)ₖ-(CH₂)ₙ-(C₆H₄)ₗ-(O)ᵣ-R group or a -(CH₂)ₘ-(C₆-₁₀)_{q}-(O)ₖ-(CH₂)ₙ-(C₆-H₁₀)ₗ-(O)ᵣ-R group,
wherein m and n each independently of the other denote the numbers 0 to 5,
k, l, q and r each independently of the others denote the number 0 or 1,
R represents a hydrogen atom, a protecting group, an optionally OR⁵-substituted C₁-C₆-alkyl radical or a CH₂-COOR⁵ group
wherein R⁵ represents a hydrogen atom, a C₁-C₆-alkyl radical or a benzyl group,
with the proviso that a direct oxygen-oxygen bond is not permitted,
R² and R³ each represent a hydrogen atom or together form a -(CH₂)ₚ-alkylene bridge
wherein p denotes the number 3 or 4, and
R⁴ represents an amino-protecting group,
**characterised in that** a nitrogen-protected aminoethyl alcohol of the general formula II 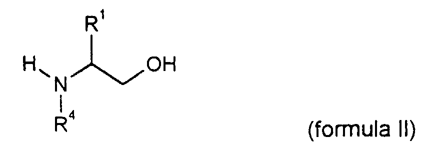 wherein R¹ and R⁴ have the meanings given,
is reacted with methanesulphonic acid chloride, tosyl chloride or trifluoroacetic acid anhydride in an organic solvent, with the addition of a base, to form the corresponding mesylate, tosylate or triflate, filtration is then carried out and the filtrate is reacted with an ethylenediamine of the general formula III 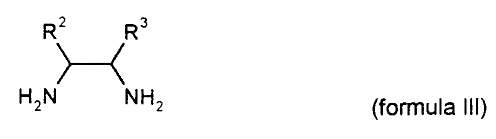 wherein R² and R³ have the meanings given,
to form the desired nitrogen-protected, carbon-substituted triamine of the general formula I.

2. Process according to claim 1, characterised in that the -(CH₂)ₘ-(C₆H₄)_{q}-(O)ₖ-(CH₂)ₙ-(C₆H₄)ₗ-(O)ᵣ-R group is an isobutyl, a benzyl, a 4-ethoxybenzyl or a 4-methoxybenzyl radical.

3. Process according to claim 1, characterised in that a benzyloxycarbonyl radical is used as the amino-protecting group R⁴.

4. Process according to claim 1, characterised in that triethylamine is used as the base.

5. Process according to claim 1, characterised in that the nitrogen-protected aminoalcohol of the formula II is reacted with methanesulphonic acid chloride.

6. Process according to claim 1, characterised in that the nitrogen-protected aminoalcohol of the formula II is reacted with toluenesulphonic acid chloride.

## Revendications

1. Procédé de préparation de diéthylènetriamines C-substituées de formule générale I, 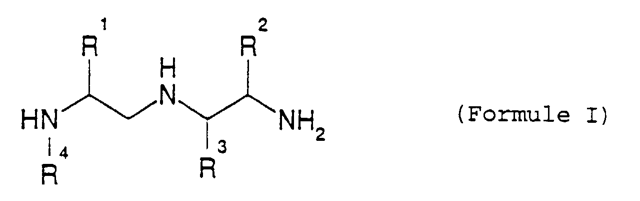 où
R¹ représente
un groupe -(CH₂)ₘ-(C₆H₄)_{q}-(O)ₖ-(CH₂)ₙ-(C₆H₄)ₗ-(O)ᵣ-R ou
un groupe -(CH₂)m-(C₆H₁₀)_{q}-(O)ₖ-(CH₂)ₙ-(C₆H₁₀)₁-(O)ᵣ-R,
où m et n, indépendamment l'un de l'autre, sont les nombres de 0 à 5,
k, l, q et r, indépendamment l'un de l'autre, valent 0 ou 1,
R représente un atome d'hydrogène, un groupe protecteur, un reste alkyle en C₁-C₆ éventuellement substitué par OR⁵, ou un groupe CH₂-COOR⁵,
avec R⁵ représentant un atome d'hydrogène, un reste alkyle en C₁-C₆ ou un groupe benzyle,
à la condition qu'une liaison directe oxygène-oxygène ne soit pas permise,
R² et R³ représentent à chacun un atome d'hydrogène ou ensemble forment un pont alkylène -(CH₂)ₚ-,
où p vaut 3 ou 4 et
R⁴ représente un groupe protecteur d'amino,
**caractérisé en ce que** l'on fait réagir l'alcool aminoéthylique protégé sur l'azote de formule générale II : 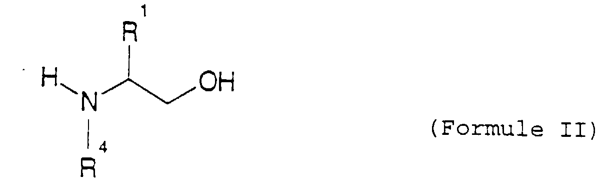 où R¹ et R⁴ ont la signification indiquée,
avec le chlorure d'acide méthanesulfonique, le chlorure de tosyle ou l'anhydride trifluoracétique dans un solvant organique, en ajoutant une base, pour obtenir un mésylate, un tosylate ou un triflate correspondant, on filtre et on fait réagir le filtrat avec une éthylènediamine de formule générale III : 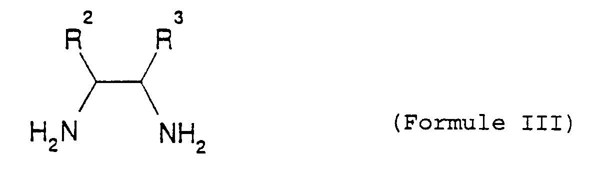 où R² et R³ ont les significations données,
pour obtenir la triamine C-substituée protégée sur l'azote, de formule générale I voulue.

2. Procédé selon la revendication 1, caractérisé en ce que le groupe -(CH₂)m-(C₆H₄)_{q}-(O)ₖ-(CH₂)ₙ-(C₆H₄)ₗ-(O)ᵣ-R représente un reste isobutyle, benzyle, 4-éthoxybenzyle ou 4-méthoxybenzyle.

3. Procédé selon la revendication 1, caractérisé en ce que R⁴, comme groupe protecteur d'amine, est un reste benzyloxycarbonyle.

4. Procédé selon la revendication 1, caractérisé en ce que l'on utilise comme base la triéthylamine.

5. Procédé selon la revendication 1, caractérisé en ce que l'on fait réagir l'aminoalcool protégé sur l'azote de formule II avec le chlorure d'acide méthanesulfonique.

6. Procédé selon la revendication 1, caractérisé en ce que l'on fait réagir l'aminoalcool protégé sur l'azote de formule II avec le chlorure d'acide toluènesulfonique.
